# EUROPEAN PATENT APPLICATION

(11) **EP 2 241 556 A1**
(43) Date of publication of application: **20.10.2010**
(21) Application number: 08857450.4
(22) Date of filing: 05.12.2008
(51) Int. Cl.: C07D 239/47, C07H 19/067

(54) **METHOD FOR THE PREPARATION OF CAPECITABINE AND INTERMEDIATES USED IN SAID METHOD**

(30) Priority: 06.12.2007 AR P070105460
(71) Applicant: Coll Farma S.L., 33428 Llanera-Asturias (ES)
(72) Inventor: BAUTISTA RODRIGUEZ, Juan, (C1425ATO) Ciudad de Buenos Aires (AR); RAVASCHINO, Esteban, (C1431EKG) Ciudad de Buenos Aires (AR); ELHALEM, Eleonora, (C1430EQA) Ciudad de Buenos Aires (AR)
(74) Representative: Robba, Pierpaolo
(86) International application number: PCT/ES2008/000766
(87) International publication number: WO 2009/071726

(57) **Abstract**

A process to obtain capecitabine compound and its pharmaceutically acceptable derivatives is hereby disclosed. Likewise, novel intermediates to be used in the preparation of capecitabine compound and its pharmaceutically acceptable derivatives are also disclosed. The procedure comprises the stage of causing a reaction of N⁴-(n-pentyloxycarbonyl))-5- fluorocytosine with (1,2,3-tri-O-acetyl-5-deoxy- α,β-D-ribofuranose.

## Description

### FIELD OF THE INVENTION

This invention refers to a process for obtaining the capecitabine compound and its pharmaceutically acceptable derivatives. Additionally, it refers to novel intermediates that can be used in the preparation of the capecitabine compound and its pharmaceutically acceptable derivatives.

### BACKGROUND OF THE INVENTION

Capecitabine is the first of a new group of fluoropyrimidines with special features. On the one hand, its oral administration is equivalent to the continuous infusion of 5-fluorouracil while it is a comfortable treatment with a higher acceptance and adherence by the sick patient. It also prevents complications and economic costs associated with IV administration. On the other hand, it provides a selective activation in the tumor which potentially enables a local treatment with the consequent improvement of the antitumor activity and reduction of systemic toxicity. (see for example: *"*Capecitabina: un quimioterápico oral en la lucha contra el cáncer de mama y colorrectal metastisico [Capecitabine: an oral chemotherapeutic in the fight against metastatic breast and colorectal cancer]", J. Calzas Rodriguez y col., FARM HOSP (Madrid), Vol. 27. N.°3, pp. 171-178, 2003.).

Capecitabine, a nucleoside analog, is a non-cytotoxic fluoropyrimidine carbamate which is administered orally and is a pro-drug of cytotoxic 5-fluorouracil (5-FU).

Capecitabine compound has the following chemical name: 5'-deoxy-5-fluoro-N⁴-pentyloxycarbonyl-cytidine (CAS Registry number: [154361-50-9]; Synonyms: Carbamic acid [1-(5-deoxy-β-D-ribofuranosyl)-5-fluoro-1,2-dihydro-2-oxo-4-pyrimidinyl] pentyl ester; Pentyl 1-(5-deoxy-β-D-ribofuranosyl)-5-fluoro-1,2-dihydro-2-oxo-4-pyrimidine carbamate).

It has the following structural formula:

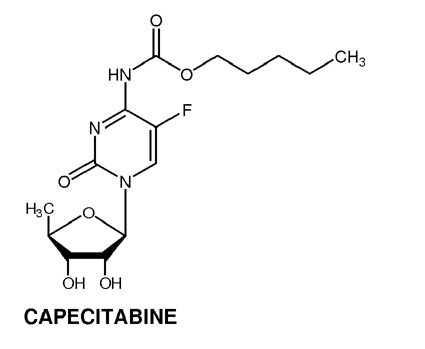

To date, synthesis routes disclosed in the literature and in patents of previous art mainly fall into some of the following three synthetic schemes:

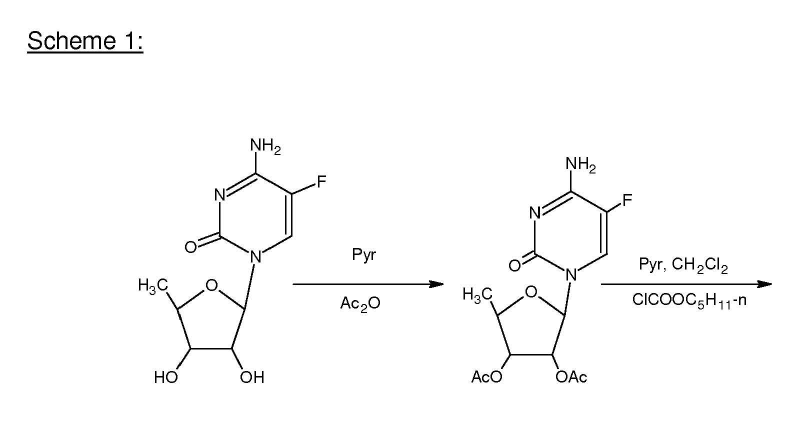

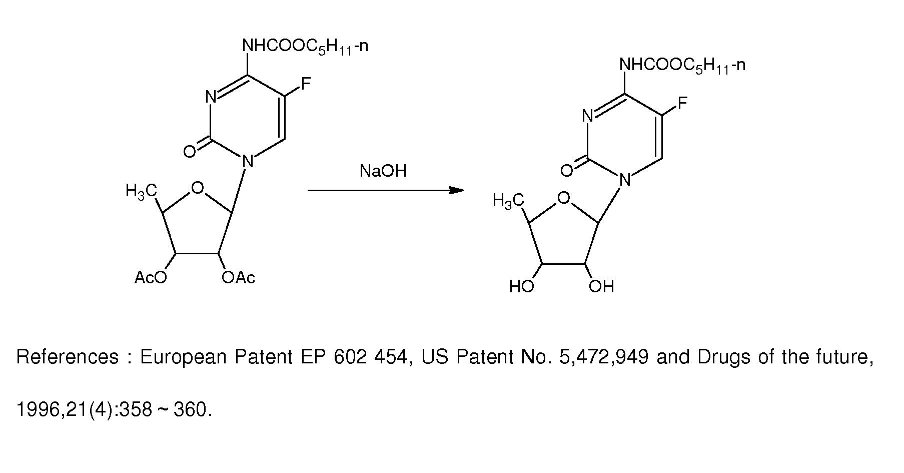

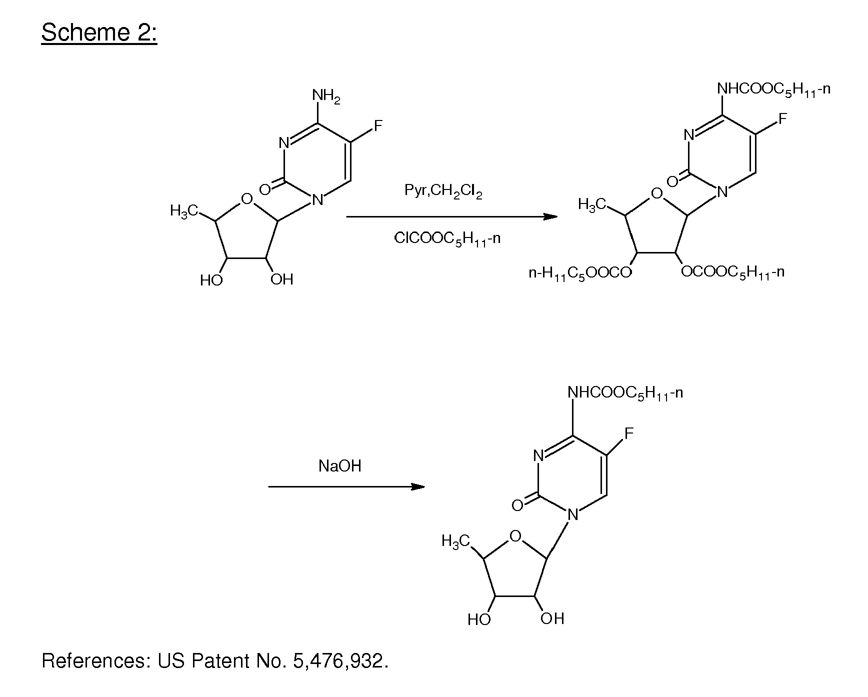

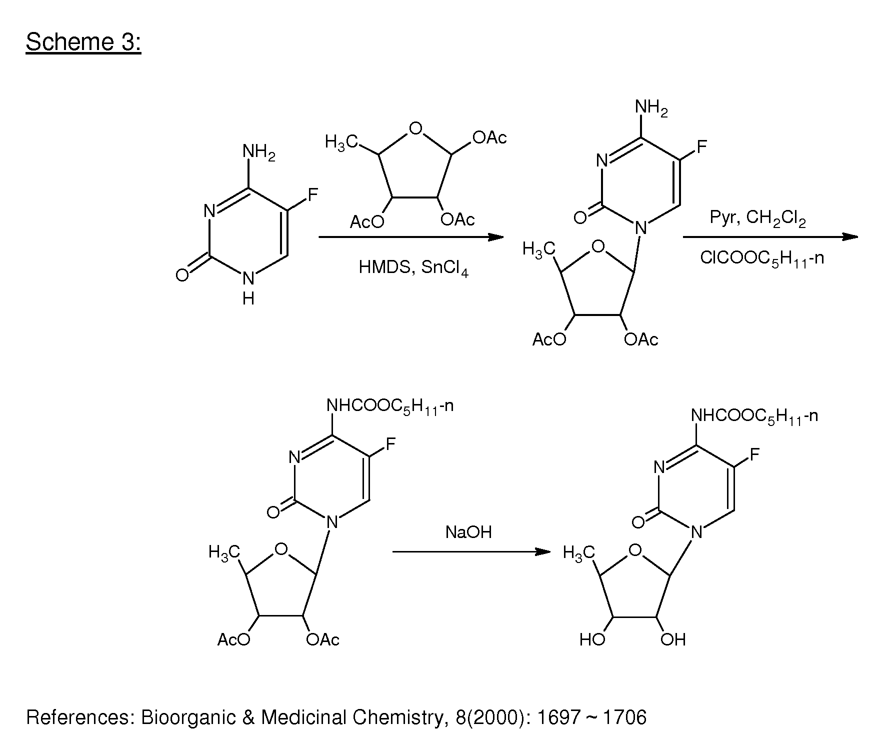

The inventors of this submission have found that, undisclosed in the previous art, the synthesis of the molecule which is aim of the capecitabine nucleoside analog may be carried out satisfactorily by employing as a key and novel step the inclusion of the heterocyclic base, derivatized as N⁴- (n-pentyloxycarbonyl))- 5-fluorocytosine (compound 2), in the derivative of 5-deoxy-D-ribose (1,2,3-tri-O-acetyl-5-deoxy-α,β-D- ribofuranose; compound 3). Thus, with the carbamate group already included, by means of a diastereoselective glycosidation reaction of said base with the derivative of 5-deoxy-D-ribose (3), the advanced and immediate precursor of the capecitabine compound may be obtained which, due to the deprotection of the corresponding protector groups, leads to the structure of the capecitabine product sought to be obtained.

### BRIEF DESCRIPTION OF THE FIGURES

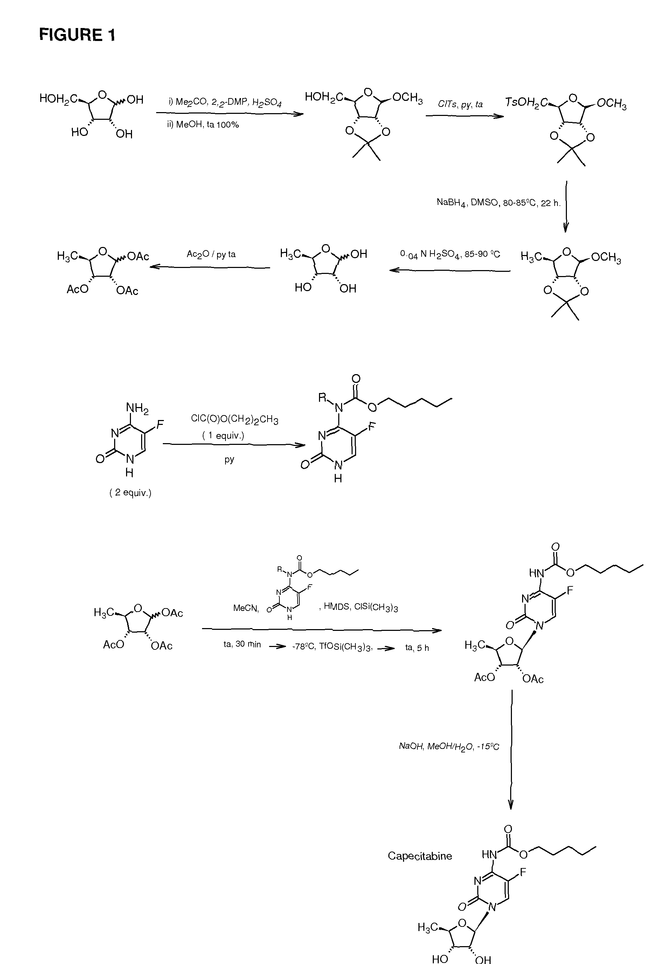

In Figure 1, a simplified scheme of a procedure for obtaining the capecitabine compound according to this invention is shown.

### DISCLOSURE OF THE INVENTION

The present invention provides a novel and enhanced procedure for obtaining capecitabine compound.

On many occasions the availability and the cost of the starting compound are the factors determining much of the difficulty and total cost of the synthesis of a given compound. In this case, the inventors of this invention have developed a procedure which used D-ribose and 5-flourocytosine, which are readily available on the market, as the starting compounds. These compounds, under adequate conditions, enable the preparation of high purity capecitabine with a good performance and a low cost.

Thus, according to the present invention, the synthesis of the target molecule of the capecitabine nucleoside analog (compound 1) may be carried out by using, as a key and novel (original) step, the inclusion of the heterocyclic base, derivatized as N⁴-(n-pentyloxycarbonyl))-5- fluorocytosine (2), in the derivate of 5-deoxy-D-ribose (1,2,3-tri-O-acetyl-5-deoxy- α,β-D-ribofuranose; compound 3). In this way, with the carbamate group already included, the diastereoselective glycosidation reaction of this base with the derivative of 5-deoxy-D-ribose (3) gives the advanced and immediate precursor of capecitabine as a result. In this way, the deprotection of the corresponding protector groups leads to obtaining the desired product (1).

One of the main advantages of the procedure to obtain capecitabine as per the invention may be found in the derivatization of the 5-fluorocytosine base to N⁴-(n-pentyloxycarbonyl)-5-fluorocytosine **(2).** Since 5-fluorocytosine is derivatized with a pentyloxycarbonyl group, it is remarkably less polar which makes it easier to handle. Indeed, compound **(2)** is significantly more soluble than the 5-fluorocytosine free base which evidences higher solubility in a great number of systems. In this way, the corresponding glycosidation reaction when the derivatized base **(2)** is used instead of the free base may be monitored much more easily using thin layer chromatography, which makes its follow up easier. On the other hand, the use of compound **(2)** reduces by one step the linear strategy usually described to obtain capecitabine. This modification in the synthesis scheme brings about both an improved global yield of the reaction and a simplification of the overall synthesis strategy.

The previous intermediary **(3)** may be obtained directly from D-ribose, a product which is readily available on the market, as illustrated for example in the retrosynthetic analysis shown in Scheme 1.

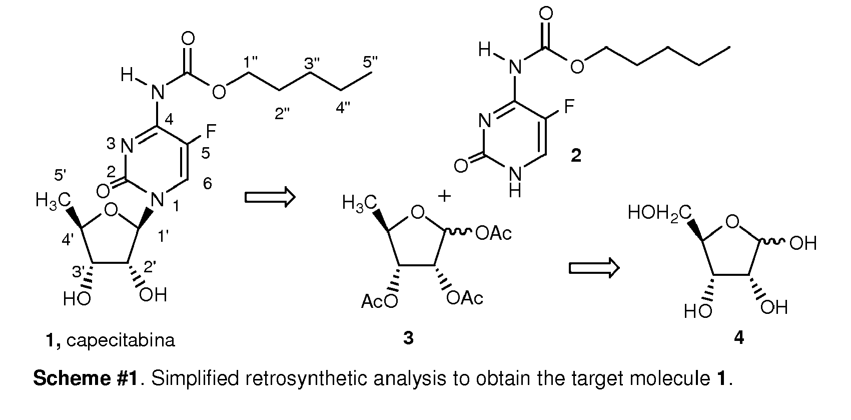

Likewise, the preparation of compound **(5),** which comprises the formation of an isipropylidene acetal group and the formation of a methyl glycoside was made in a reaction carried out in a single "one pot" step. This reaction was carried out in the multigram scale starting from up to 20 grams of D-ribose obtaining the desired compound **(5)** without the need of purification with theoretical yield. The existing data in the bibliography support the feasibility of this method. ^{[1-5]} The resulting product was treated with excess of tosyl chloride in pyridine at 0°C which gives the corresponding tosylate **(6)** with a very good yield. This product evidenced a spectrum which coincides with that of the desired product. ^{[6]} The reduction of **(6)** was carried out following the method described in the literature ^{[7]} treating this compound with sodium borohydride (4 moles per mole of starting product) in dimethyl sulfoxide at 85°C for 13 hours which results in compound **(7).**

The reaction of deprotection of methylglycoside **(7)** together with the removal of the isopropylene group followed by peracetylation was solved adequately. The potential inconvenients arising from this reaction are described in the bibliography, among which are epimerization in position C-2 to form the corresponding arabin derivative when the acetylation reaction is done together with the corresponding hydrolysis ^{[7-12]}.

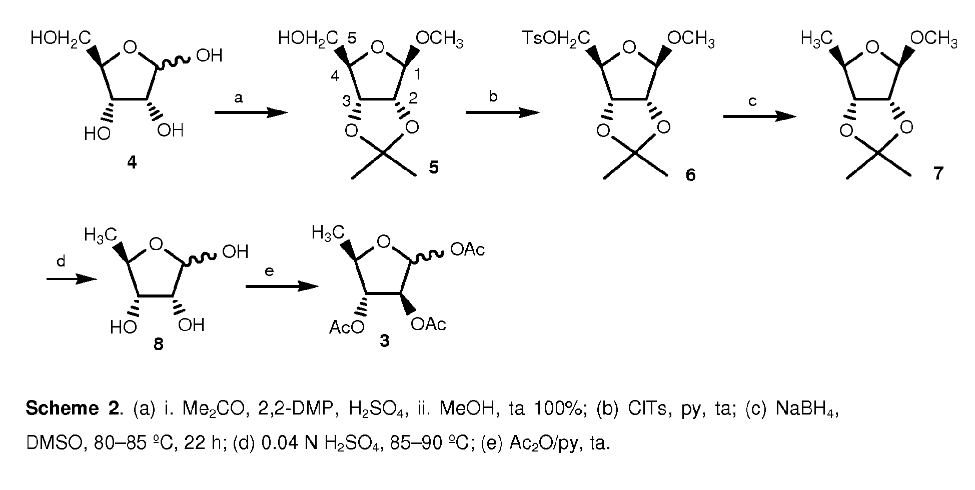

The previous precursor **(3)** may be obtained by sequential treatment, first with sulfuric acid 0.04 N at 80-90 °C to obtain **(8)** followed by a treatment with acetic anhydride in pyridine. The structural confirmation may be obtained, for example, by the analysis of the coupling constants of the proton spectrum of structurally related compounds. ^{13, 14} Although there was a method described in the literature for the preparation of compound **(3)** mentioned above^{[15]}, up to the moment there were no spectroscopic data published to determine without ambiguity the obtaining of the desired product.

Once the compound **(3)** is available, the coupling with the adequately derivatized base **(2)** is done, which may be prepared by using the treatment with 5-fluorocytosine **(9)** with chloroformiate of n-pentyl in pyridine. Because of the tendency of the heterocyclic base to suffer diacylations, it was found that it is deemed convenient to work with an amount of acylating agent less than the stechiometric requirement, since the free base **(9)** may be recovered once the reaction is finished (Scheme 3).

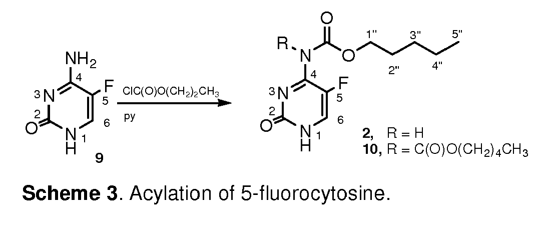

Once the functionalized base **(2)** is obtained, it is coupled with the heterocyclic base by means of an appropriate glycosidation reaction. ^{[16-19]} In that manner, a suspension of **2 and 3** in anhydrous acetonitrile is treated with hexamethyldisilazane anhydrous and chlorotrimethylsilane. Once the reaction mixture is cooled at approximately -78°C, trimethylsilane triflate is added. Once pouring is completed it is taken to ambient temperature and stirred for approximately five hours to obtain the desired product. **(11).** ^{[20]}

The structural confirmation of the intermediary **(11)** was carried out by acetylating capecitabine standard, which was turned into **(11)** by treating it with acetic anhydride in pyridine. The spectrums of the acetylation product of capecitabine and **(11)** coincided, thus confirming the structure of the peracetylated sugar **(3).**

The hydrolysis of the intermediary product **(11)** by treatment with sodium hydroxide in methanol enables obtaining capecitabine 1 (Scheme 4). ^{[21]}

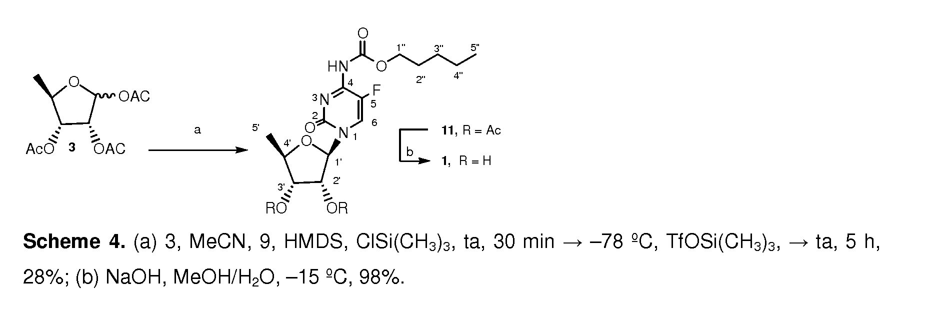

According to laboratory tests, the reaction scheme of the invention enables to drastically reduce the elaboration costs of the product while it permits to obtain a product of excellent quality and stability at the same time.

### EXEMPLARY EMBODIMENTS

The following examples are not intended as limitation and are included only to illustrate a manner to use the present invention in the practice.

### 1) Obtaining Methyl 2,3-O-isopropyliden-β-D-ribofuranoside (5)

A suspension of D-ribose **(4**, 10g) in acetone (80 mL) cooled to 0°C was added drop by drop to 2,2-dimethyoxypropane (20 mL) with perchloric acid 70% (4 mL). It was stirred for two hours at ambient temperature and then methanol (14 mL) was added and the mixture was stirred at ambient temperature for 16 hours. A saturated solution of K₂CO₃ (10 mL) was added and the mixture was partitioned in water (150 mL) and CH₂Cl₂ (70 mL). The aqueous phase was extracted with CH₂Cl₂ (3 x 70 mL). The combined organic phases were dried (Na₂SO₄) and the solvent was evaporated to obtain 13,5 g of compound 5 as a colorless syrup. This same reaction was carried out starting from 20 g of D-ribose, obtaining the desired product with a 100% yield.

### Methyl 2,3-O-isopropyliden-5-O-tosyl-β-D-ribofuranoside (6)

A solution of methyl 2,3-O-isopropyliden-β-D-ribofuranoside (5; 22,6 g, 0,11 mol) in pyridine (25 mL) cooled at 0°C was treated with tosyl chloride (31,6 g, 0,17 mol) added in 4 similar parts. The reaction mixture was stirred for 3,5 hours at 0°C. Then it was poured into a mixture of 250 g of ice and 250 g of water. The ice was let to melt by stirring and the mixture was filtered. The filtrate was washed with cold water (2 x 100 mL) and it was dried with the desiccator. The compound 6 was obtained as a white crystalline solid (35,6 g, 89% yield starting from d-ribose) ¹H RMN (CDCl₃, 500 MHz) δ 1.29 (s, 3H, CCH₃), 1.45 (s, 3H, CCH₃), 2.46 (s, 3H, PhCH₃), 3.23 (OCH₃), 4.00 (dd, J = 10.0, 6.8 Hz, 1H, H-5'ₐ), 4.03 (dd, J = 9.9, 7.6 Hz, 1H, H-5'_{b}), 4.31 (t, J = 7.2 Hz, 1H), 4.53 (d, J = 5.9 Hz, 1H), 4.60 (d, J = 5.9 Hz, 1H), 7.36 (d, J = 8.1 Hz, 2H, H-3"), 7.81 (d, J = 8.3 Hz, 2H, H-2").

### Methyl 2,3-0-isopropyliden-5-deoxy- β -D-ribofuranoside (7)

A solution of compound **6** (5.12 g, 14.3 mmol) in DMSO (40 mL) was treated with NaBH₄ (2.16 g, 57.1 mmol) and the mixture was stirred at 85°C for 13 hours. The reaction ended by adding a saturated solution of NH₄Cl (200 mL). Then CH₂Cl₂ (200 mL) was added. The organic phase was washed with a saturated solution of sodium chloride (4 x 100 mL). It was dried (Na₂SO₄) and the solvent was evaporated. The residue was purified by column chromatography using CH₂Cl₂ as eluant to obtain the desired product 7 with a 100% yield.

### 1,2,3-Tri-O-acetyl-5-deoxy-β-D-ribofuranose (3)

Methyl 2,3-O-isopropyliden- 5-deoxy-β-D-ribofuranoside (207 mg, 1,09 mmol) was suspended in H₂SO₄ 0.04 N (2 mL) and was taken to 80-90°C. The mixture resulting from the reaction was stirred at that temperature for 3.5 hours. It was neutralized with NaHCO₃ (s) and the solvent was evaporated at a reduced pressure. A solid white crystalline residue was obtained, which was resuspended in pyridine (3 mL) and Ac₂O (1 mL) was added at ambient temperature. It was stirred for 20 hours at ambient temperature and the solvent was evaporated at reduced pressure. The obtained residue was purified by means of column chromatography (silica gel) using a mixture of hexane-ethyl acetate (4:1) as eluent. A mixture of the anomers α and β of 1,2,3-tri-O-acetyl-5-deoxy-D-ribofuranoside was obtained. Anomer β was favored with a 40% yield, starting from methyl 2,3-O-isopropyliden-5-deoxy- β-D-ribofuranoside, as a colorless oil, which partly crystallized after storing it at -15°C.

This reaction was repeated starting from 1 g of methyl 2,3-O-isopropyliden-5-deoxy-β-D-ribofuranoside, slightly changing the reaction times: 4 hours for hydrolysis and 30 hours for acetylation. The desired compound was obtained as a mixture of anomers α and β, anomer β being favored with an overall yield of 49% starting from methyl 2,3-O-isopropyliden-5-deoxy-β-D-ribofuranoside after purification using column chromatography.

**Anomer β**: H RMN (CDCl₃, 500 MHz) δ 1.38 (d, J = 6.5 Hz, 3H, H-5), 2.08 (s, 3H, COCH₃), 2.10 (s, 3H, COCH₃), 2.12 (s, 3H, COCH₃), 4.28 (p, J = 6.5 Hz, 1H, H-4), 5.10 (dd, J = 6.7, 4.9 Hz, 1H, H-3), 5.34 (dd, J = 4.8, 0.9 Hz, 1H, H-2), 6.11 (d, J = 0.8 Hz, 1H, H-1); ¹³C RMN (CDCl3, 500 MHz) δ 19.72 (C-5), 20.48 (COCH₃), 20.51 (COCH₃), 21.06 (COCH₃), 74.63, 74.95, 77.97 (C-4), 98.27 (C-1), 169.32 (COCH₃), 169.47 (COCH₃), 169.89 (COCH₃).

### N⁴-(n-Pentyloxycarbonyl))-5-fluorocytosine (2); bis-(N⁴,N⁴-(n-Pentyloxycarbonyl))- 5-fluorocytosine (10)

N-pentyl chloroformate (325 mg, 2,16 mmol) was added drop by drop at ambient temperature to a suspension of 5-fluorocytosine **(9;** 200 mg, 1,55 mmol) in pyridine (1 mL). The reaction mixture was stirred for 16 hours at ambient temperature. Then methanol (5 mL) was added and evaporated at a reduced pressure. A white solid residue was obtained which contained a mixture of N⁴-(n-pentyloxycarbonyl)-5-fluorocytosine **(2),** bis-(N⁴,N⁴-(n-pentyloxycarbonyl))- 5-fluorocytosine **(14)** and 5-fluorocytosine **(10).** The product was purified by means of column chromatography (silica gel) eluting with mixtures which ranged from pure ethyl acetate to ethyl acetate-methanol (4:1). 92 mg of N⁴-(n-pentyloxycarbonyl))-5-fluorocytosine **2** were obtained (24% yield).

The previous reaction was repeated starting from 1.00 g of 5-fluorocytosine. 220 mg of the desired product were obtained (10% yield) due to the difficulty in the purification stage caused by the high polarity and insolubility in organic solvents, inherent in these compounds. With the aim of obtaining enough quantity of N⁴-(n-pentyloxycarbonyl)-5-fluorocytosine to test the coupling with the peracetylated sugar **3,** this reaction was repeated starting from 2.00 g of **12,** using less amount of the required stecheometricaly of n-pentyl chloroformate (1.17 g, 7.75 mmol) thus preventing diacylation of the base. With the same aim, the reaction was carried out at 0ºC. Nevertheless, no reaction was observed at this temperature. When taking the mixture to ambient temperature for 16 hours again a mixture of mono **(2)** diacylated **(10)** product and base without reaction **(9)** was obtained, the latter being the majority compound. This mixture was purified by means of reverse phase column chromatography (RP-18) eluting with a water-methanol mixture (3:2). This purification permitted to easily eliminate the product of diacylation. The mixture of the remaining two components was separated by using column chromatography (silica gel) eluting it with mixtures ranging from pure ethyl acetate to ethyl acetate 4:1. After successive chromatography runs 380 mg of N⁴-(n-pentyloxycarbonyl)-5-fluorocytosine 10 with a 20% yield were obtained. **Compound 2**: ¹H RMN (500 MHz, DMSO-d₆) δ 0.85 (m, 3H, H-5'), 1.28 (m, 4H, H-3', H-4'), 1.57 (m, 2H, H-2'), 4.40 (t, J = 6.5 Hz, 2H, H-1'), 7.94 (d, J = 5.7 Hz, 1H, H-1), 11.09 (s wide, 1H, NHCO) ; ¹³C RMN (75 MHz, CDCl₃) δ 13.14 (C-5'), 21.22 (C-4'), 26.91 (C-3'), 27.33 (C-2'), 64.73 (C-1'), 128.90 (s wide, C-6), 137.66 (d, J = 141.0 Hz, C-5), 149.90 (NC(O)O), 152.20 (d, J = 9.8 Hz, C-4). **Compound 10:** ¹H RMN (500 MHz, CDCl₃) δ 0.89 (t, J = 7.0 Hz, 6H, H-5'), 1.31 (m, 8H, H-3', H-4'), 1.67 (p, J = 7.0 Hz, 4H, H-2'), 4.27 (t, J = 6.7 Hz, 4H, H-1'), 8.13 (d, J = 3.4 Hz, 1H, H-1); ¹³C RMN (125 MHz, CDCl₃) δ 13.82 (C-5'), 22.08 (C-4'), 27.66 (C-3'), 27.93 (C-2'), 68.44 (C-1'), 134.58 (d, J = 30.0 Hz, C-6), 142.27 (d, J = 247.1 Hz, C-5), 150.13 (NC(O)O), 155.47 (d, J = 13.6 Hz, C-4), 157.03 (C-2).

### 2',3'-Di-O-acetyl-5'-deoxy-5-fluoro-N⁴⁻(n-pentyloxycarbonyl)cytidine (11)

A solution of 1,2,3-tri-O-acetyl-5-deoxy-D- ribofuranoside (107 mg, 0,41 mmol) in anhydrous acetonitrile (3 mL) was treated with N⁴-(n-pentyloxycarbonyl)-5-fluorocytosine (100 mg, 0,41 mmol) under an argon atmosphere. The resulting suspension was treated with hexamethyldisilazane anhydrous (87 µL, 0,41 mmol) and chlorotrimethylsilane (210 µL, 1,64 mmol). It was stirred at ambient temperature for 30 minutes. The reaction mixture was cooled at -78°C and trimethylsilyl triflate (87 µL, 0,49 mmol) was added. It was taken one more time to ambient temperature and it was stirred for 5 hours. It was diluted with dichloromethane (50 mL) and it was washed with water (2 x 20 mL). The organic phase was dried (Na₂SO₄) and the solvent was evaporated. The product was purified by means of column chromatography (silica gel). A mixture of hexane-ethyl acetate (4:1) was used as elution which gave 51 mg (28% yield) of the desired product as a colorless oil. ¹H RMN (CDCl₃, 500 MHz) δ 0.91 (t, J = 7.1 Hz, 3H, H-5"), 1.36 (m, 4H, H-3", H-4"), 1.47 (d, J = 6.5 Hz, 3H, H-5'), 1.71 (p, J = 7.0 Hz, 2H, H-2"), 2.10 (s, 3H, COCH₃), 2.11 (s, 3H, COCH₃), 4.17 (t, J = 5.6 Hz, 2H, H-1 "), 5.01 (t, J = 5.5 Hz, 1H, H-), 5.30 (t, J = 5.4 Hz, 1H, H-) 5.95 (d, J = 4.2 Hz, 1H, H-1'), 7.42 (s, 1H, ), 12.01 (s, 1H); ¹³C RMN (CDCl₃, 125 MHz) δ13.85, 18.60 (C-5'), 20.32 (COCH₃), 20.43 (COCH₃), 22.23 (C-4"), 27.85 (C-3"), 28.17 (C-2"), 66.59 (C-1"), 72.91, 73.88, 78.20, 87.96, 123.38, 123.65, 138.93, 146.18, 153.09, 153.25, 163.44, 169.56 (COCH₃), 169.63 (COCH₃).

### 5'-Deoxy-5-fluoro-N⁴-(n-pentyloxycarbonyl)cytidine, capecitabine (1)

A solution of NaOH (2 mg, 0.05 mmol) in water (0.5 mL) was slowly added to a solution of 2',3'-di-O-acetyl-5'-deoxy-5-fluoro-N⁴- (n-pentyloxycarbonyl)cytidine (20 mg, 0.045 mmol) in methanol (1.0 mL) at -15 ºC. The reaction mixture was stirred at that temperature for 20 hours. It was taken to pH = 5 with HCl 5% keeping the temperature at -15°C and it was partitioned in CH₂Cl₂ (10 mL) and saturated solution of NaCl (5 mL). The organic phase was washed with a saturated solution of NaCl (5 mL), it was dried (Na₂SO₄), and the solvent was evaporated. 16 mg of capecitabine (98% yield) were obtained as a crystalline white solid. ¹H RMN (CDCl₃-DMSO-d₆, 500 MHz) δ 0.91 (t, J = 6.9 Hz, 3H, H-5"), 1.36 (m, 7H, H-3", H-4", H-5'), 1.67 (quint, 2H, J = 6.6 Hz, H-2"), 3.69 (s a, 1H, H-3'), 4.03 (s a, 1H, H-4'), 4.09 (m, 1H, H-2'), 4.14 (s a, 2H, H-1 "), 4.80 (s a, 1H, 3'-OH), 5.44 (s a, 1H, 2'-OH), 5.70 (d, J = 2.3 Hz, H-1'), 7.85 (s a, 1H, H-6), 10.22 (s a, 0.61H, NH), 11.77 (s a, 0.39H, NH).

### REFERENCES

[1] Schmidt, O. T. Isopropylidene Derivatives. Methods Carbohydr. Chem. 1963, II, 318-325.
[2] de Belder, A. N. Cyclic acetals of the Aldoses and Aldosides Adv. Carbohydr. Chem. 1965, 20, 219-302.
[3] Rodriguez, J. B. Chiral 1,4-Dicarbonyl-2,3-O-lsopropylidene Derivatives. Rapid Racemization on Standing. Tetrahedron 1999, 55, 2157-2170.
[4] Hough, L.; Jones, J. K. N.; Mitchell, D. L. The Preparation of Some Derivatives of D-Ribono-1→4-Lactone and D-Ribitol. Can. J. Chem. 1958, 36, 1720-1728.
[5] Camps, P.; Cardellach, J.; Font, J.; Ortuño, R. M.; Ponsati, O. Studies on structurally simple ,β-butenolides―II : (-)-(S)-γ-hydroxymethyl-α,β-butenolide and derivatives from D-ribonolactone efficient synthesis of (-)-ranunculin. Tetrahedron 1982, 38, 2395-2402.
[6] Sairam, P.; Puranik, R.; Rao, B. S.; Swamy, P. V.; Chandra, S. Synthesis of 1,2,3-tri-O-acetyl-5-deoxy-D-ribofuranose from D-ribose. Carbohydr. Res. 2003, 338, 303-306.
[7] Lerner, L. M. Preparation of 9-(5-Deoxy-α-D-arabinofuranosyl)-adenine from D-Ribose, J. Org. Chem. 1978, 43, 161-163*.*
[8] Sowa, W. Epimerization of Monosaccharides Under Acetolysis Conditions. Can. J. Chem. 1971, 49, 3292-3298.
[9] Sowa, W. Epimerization of Monosaccharides in Acetic Acid and Acetic Anhydride. Synthesis of D-Altrose. Can. J. Chem. 1972, 50, 1092-1094.
[10] Chittenden, G. J. F. Rearrangement of some D-ribose and D-lyxose derivatives under acetolysis conditions. Carbohydr. Res. 1972, 22, 491-493.
[11] Boon, P. J.; Schwartz, A. W.; Chittenden, G. J. F. Acetolysis-epimerisation studies : Part 1. Acetolysis of L-rhamnose and some derivatives: formation of L-quinovose (6-deoxy-L-glucose. Carbohydr. Res. 1973, 30, 179-182.
[12] Lerner, L. M. Interconversions of hexofuranosyl nucleosides. IV. Synthesis of nucleosides derived from 6-deoxy-L-glucose. J. Org. Chem. 1972, 37, 4386-4391.
[13] Gandolfi-Donadio, L.; Gallo-Rodriguez, C.; de Lederkremer, R. M. Facile synthesis of α-D-Araf-(1→5)-D-Galf, the linker unit of the arabinan to the galactan in Mycobacterium tuberculosis. Can. J. Chem. 2006, 84, 486-491.
[14] Mariño, K.; Marino, M. C.; Lima, C.; Baldón, L.; de Lederkremer, R. M. The First Chemical Synthesis of UDP[6-3H]-a-D-galactofuranose. Eur. J. Org. Chem. 2005, 2958-2964.
[15] D'Sousa, R.; Kiss, J. Patent EP 0 021 231, 1981.
[16] Kim, H. O.; Schinazi, R. F.; Shanmuganathan, K.; Jeong, L. S.; Beach, W.; Nampalli, S.; Cannon, D. L.; Chu, C. K. L- -(2S,4S)- and L-α-(2S,4R)-Dioxolanyl Nucleosides as Potential Anti-HIV Agents: Asymmetric Synthesis and Structure-Activity Relationships. J. Med. Chem. 1993, 36, 519-528.
[17] Jeong, L. S.; Schinazi, R. F.; Beach, J. W.; Kim, H. O.; Nampalli, S.; Shanmuganathan, K.; Alves, A. J.; McMillan, A.; Chu, C. K.; Mathis, R. Asymmetric Synthesis and Biological Evaluation of β-L-(2R,5S)- and -L-(2R,5R)-1,3-Oxathiolane-Pyrimidine and -Purine Nucleosides as Potential Anti-HIV Agents. J. Med. Chem. 1993, 36, 181-195.
[18] Jeong, L. S.; Schinazi, R. F.; Beach, J. W.; Kim, H. O.; Shanmuganathan, K.; Nampalli, S.; Chun, M. W.; Chung, W. -K.; Choi, B. G.; Chu, C. K. Structure-Activity Relationships of β-D-(2S,5R)- and α-D-(2S,5S)-1,30xathiolanyl Nucleosides as Potential Anti-HIV Agents. J. Med. Chem. 1993, 36, 2627-2638.
[19] Kim, H. O.; Schinazi, R. F.; Nampalli, S.; Shanmuganathan, K.; Cannon, D. L.; Alves, A. J.; Jeong, L. S.; Beach, J. W.; Chu, C. K. 1,3-Dioxolanylpurine Nucleosides (2R,4R) and (2R,4S) with Selective Anti-HIV-1 Activity in Human Lymphocytes. J. Med. Chem. 1993, 36, 30-37.
[20] Secrist, III, J. A. Preparation of Thioarabinofuranosyl Compounds and Use *Thereof.* U S Patent 6,576,621.
[21] Shimma, N.; Umeda, I.; Arasaki, M.; Murasaki, C.; Masabuchi, K.; Kohchi, Y.; Miwa, M.; Ura, M.; Sawada, N.; Tahara, H.; Kuruma, I.; Horii, I.; Ishitsuka, H. The Design and Synthesis of a New Tumor-Selective Fluoropyrimidine Carbamate, Capecitabine. Bioorg. Med. Chem. 2000, 8, 1697-1700.

## Claims

1. A process for obtaining capecitabine **characterized** because it comprises reacting the compound N⁴-(n-pentyloxycarbonyl))-5- fluorocytosine **(2)** with the compound (1,2,3-tri-O-acetyl-5-deoxy- α,β-D-ribofuranose **(3)**.

2. A process for obtaining capecitabine according to claim 1, **characterized** because 1,2,3-tri-O-acetyl-5-deoxy- α,β-D-ribofuranose **(3)** is obtained from D-ribose.

3. A process for obtaining capecitabine according to claim 1, **characterized** because it further comprises to obtain the compound methyl 2,3-O-isopropyliden- β-D-ribofuranoside **(5)** from D-ribose.

4. A process for obtaining capecitabine according to the previous claim, **characterized** because it further comprises to react the compound methyl 2,3-O-isopropyliden- β-D-ribofuranoside **(5)** with tosyl chloride to obtain the compound methyl 2,3-O-isopropyliden-5-O-tosyl-β-D-ribofuranoside **(6)**.

5. A process for obtaining capecitabine according to the previous claim, **characterized** because it further comprises the reduction of compound methyl 2,3-O-isopropyliden-5-O-tosyl- β-D-ribofuranoside **(6)**, to obtain the compound methyl 2,3-0-isopropyliden-5-deoxy- β -D-ribofuranoside **(7)**.

6. A process for obtaining capecitabine according to the previous claim, **characterized by** reacting the compound methyl 2,3-O-isopropyliden-5-deoxy-β -D-ribofuranoside **(7)** with sulfuric acid followed by a treatment with acetic anhydrous in pyridine in order to obtain compound 1,2,3-tri-O-acetyl-5-deoxy-α,β-D-ribofuranose **(3)**.

7. A process for obtaining capecitabine according to claim 1, **characterized** because it further comprises to react 5-fluorocytosine **(9)** in pyridine with n-pentyl chloroformate to obtain N⁴-(n-pentyloxycarbonyl))-5- fluorocytosine **(2).**

8. A process for obtaining capecitabine according to claim 1 **characterized** because it further comprises to react 1,2,3-tri-O-acetyl-5-deoxy-D-ribofuranoside in acetonitrile anhydrous with N⁴-(n-pentyloxycarbonyl)-5-fluorocytosine under an argon atmosphere and treating the suspension thus obtained with hexamethyldisilazane anhydrous, chlorotrimethylsilane and trimethylsilyl triflate to obtain compound 2',3'-Di-O-acetyl-5'-deoxy-5-fluoro-N⁴-(n-pentyloxycarbonyl)cytidine **(11).**

9. A process for obtaining capecitabine according to the previous claim, **characterized** because it further comprises the hydrolysis of compound 2',3'-Di-O-acetyl-5'-deoxy-5-fluoro- N⁴-(n-pentyloxycarbonyl)cytidine **(11)** to thus obtain the capecitabine compound.

10. A compound to be used as intermediate in the procedure of claim 1, **characterized** because it is the compound N⁴-(n-pentyloxycarbonyl))-5-fluorocytosine **(2).**

11. A process for obtaining capecitabine according to the previous claim, **characterized** because it corresponds with the following synthesis scheme:
